# EUROPEAN PATENT APPLICATION

(11) **EP 1 908 405 A2**
(43) Date of publication of application: **09.04.2008**
(21) Application number: 07253884.6
(22) Date of filing: 01.10.2007
(51) Int. Cl.: A61B 6/00

(54) **Dual-radiation type mammography apparatus and breast imaging method using the mammography**

(30) Priority: 02.10.2006 KR 20060096980
(71) Applicant: Wonkwang University Center for Industry - Academy Cooperation, Ikan-si, Jeollabuk-do 570-749 (KR)
(72) Inventor: Yoon, Kwon-ha, Jeollabuk-do 570-972 (KR)
(74) Representative: Neobard, William John

(57) **Abstract**

A dual-radiation type mammography apparatus for examining a breast by taking a breast image using x-ray beams is provided. The mammography apparatus includes an x-ray generating unit (10) generating the x-ray beams, a first collimator (20) provided with transmission holes (22) through which a solid angle beam generated from the x-ray beams radiated from the x-ray generating unit, in the course of which the solid angle beam is changed into a plurality of beams each having a predetermined size, a plurality of multi-layered filter units (30) that filters off some of the beams passing through the holes of the first collimator to deflect only beams in a first specific energy band, a breast fixing unit (50) fixing the breast B that will be scanned by the beams deflected by the multi-layered filter units, and a plurality of image output units (60) provided for the respective beams scanning the breast fixed by the breast fixing unit and reforming two-dimensional images formed by the beams deflected at different angles into a three-dimensional image.

## Description

The present invention relates to a dual-radiation type mammography. Embodiments relate to a dual-radiation type mammography apparatus that can easily examine the breast by taking a breast image by scanning the breast using a plurality of fan beams that are formed from beams radiated from an x-ray generating unit and radiated at different angles and by reforming the image as a three-dimensional breast image and that can accurately determine a location and size of lesion.

Generally, a mammography apparatus (or referred to as "breast cancer examining apparatus") uses a rotating target type x-ray generating device. Molybdenum or rhodium is usually used as a target material. A maximum tube voltage applied to an anode of the x-ray generating device is 40kVp. Electron beams radiated from a filament of an x-ray generating unit collide with the anode, by which x-ray beams are generated. The breast image for examining is captured using the x-ray light.

In this case, beams in a specific energy band except for an energy band of 15-20keV within which a contrast between a soft tissue and a tumor in an x-ray image of the breast is excellent does not affect on the actual image. Particularly, beams in a relatively low energy band of 8-15keV are mostly absorbed in the breast and thus increases the x-ray exposure dose to the patient. Beams in a high energy band above 20keV causes blurring of the image due to Compton scattering.

Meanwhile, in order to minimize the x-ray exposure to the breast by the relatively low energy band, a filter such as an aluminum filter, a molybdenum filter, or a rhodium filter is used to filter off beams in a specific energy band. In this case, since the filter may partly filter x-rays, e.g., 17.48 keV(Mo Target), which is effective for the image, the x-ray radiation time must be increased and the quality of the image is deteriorated.

An imaging apparatus using a monochromatic beam of a specific x-ray at the Bragg angle through a silicon signal crystal has been recently developed. In this case, since a band width of a specific energy is narrow, the dose of the x-ray exposure and the scattering can be reduced. However, since the photon flux is too weak, the image must be taken for a long time.

Meanwhile, a latest mammography apparatus takes a breast image by radiating the solid angle x-ray from an x-ray generating unit to an entire region of the breast using a collimator or takes the breast image by scanning the breast using a single pan beam formed by a special collimator.

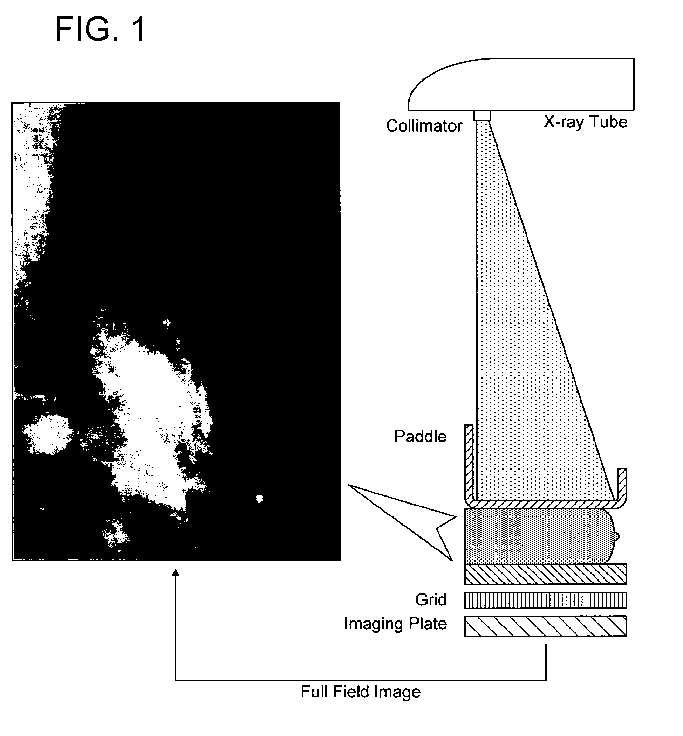

FIG. 1 is a schematic view of a prior art mammography apparatus. Referring to FIG. 1, a sold angle x-ray is used to take an image of the overall breast through one x-ray scanning. This has an advantage of reducing the scanning time. However, since the x-rays of the lower energy band (8-15keV) are absorbed in the breast, the exposure dose increases and the resolution of the image is deteriorated.

To overcome the above problems, a mammography apparatus using fan beams has been proposed. In this mammography apparatus using the fan beams, the x-rays generated from an x-ray generating unit pass through a collimator so that only the x-rays required for taking the image can be directed to the breast. Therefore, the x-ray dose of this mammography apparatus is less than that of the solid angle mammography apparatus and the resolution of the image can be improved.

However, in the mammography apparatus using the fan beams, the x-ray generating unit and the image detecting unit are located on a vertical line. Therefore, when lesions overlap on the vertical line, the image is taken as having one lesion. Therefore, it is difficult to accurately identify the locations and sizes of the lesions. Therefore, the mammography apparatus using the fan beams is simply used to identify if there is the breast cancer.

To solve the above problems, the image of the breast is further taken after turning the x-ray generating unit and the image detecting unit leftward or rightward by 15-30°. By doing this, the locations of the lesions overlapping on the vertical line can be identified. However, the images are independently taken and combined to each other to look for the locations of the lesions. However, the combining process of the images is complicated.

In addition, in order to take a biopsy of the lesions by extracting the tissues of the lesions, the photographing must be done at least three times and thus the exposure dose increases. In addition, the photographing time increases. Further, the locations of the lesions are usually identified by feeling from the two-dimensional image or by additionally taking an image while inserting a lesion extracting needle little by little.

However, in order to perform the above-describe method, the patient's breast are pressed for a long time. This exacerbates the patient's pain and increases the exposure dose, thereby inducing a secondary accident. Furthermore, since the two-dimensional image is used to identify the locations and sizes of the lesions, this method cannot assist the user to accurately identify the locations and sizes of the lesions.

Embodiments provide a dual-radiation type mammography apparatus that can easily examine the breast by taking an image by scanning the breast using a plurality of fan beams that are formed from beams radiated from an x-ray generating unit and radiated at different angles and reforming the image as a three-dimensional breast image and can accurately determine a location and size of lesion, thereby enabling the data collection for maximizing treatment efficiency, minimizing the exposure dose to a patient by reducing the image taking time, and minimizing the patient's pain.

According to an aspect of the present invention, a dual-radiation type mammography apparatus for examining a breast by taking a breast image using x-ray beams includes an x-ray generating unit generating the x-ray beams; a first collimator provided with transmission holes through which a solid angle beam generated from the x-ray beams radiated from the x-ray generating unit, in the course of which the solid angle beam is changed into a plurality of beams each having a predetermined size; a plurality of multi-layered filter units that filters off some of the beams passing through the holes of the first collimator to deflect only beams in a first specific energy band; a breast fixing unit fixing the breast B that will be scanned by the beams deflected by the multi-layered filter units; and a plurality of image output units provided for the respective beams scanning the breast fixed by the breast fixing unit and reforming two-dimensional images formed by the beams deflected at different angles into a three-dimensional image.

The dual-radiation type mammography apparatus may further include a second collimator that uniformly maintaining a width of each of the beams when the beams deflected by the multi-layered filter units scan the breast. In addition, the breast fixing unit may include a pressure sensor for uniformly controlling fixing pressure pressing the breast.

According to another aspect of the present invention, a breast imaging method for examining a breast by taking a breast image using x-ray beams includes fixing the breast by applying predetermined pressure to the breast; forming a plurality of beams by allowing beams in a specific energy band among the solid angle beam generated from an x-ray generating unit to pass and allowing rest beams to be blocked; filtering some of the beams passing through the holes of the first collimator and deflecting only beams in a specific energy band at different angles; scanning the breast using the beams deflected at different angles; and capturing two-dimensional scan images formed by the beams deflected at the different angles and reforming the two-dimensional scan images into a three-dimensional image through a geometrical calculation.

The breast imaging method may further include maintaining uniformly a width of each of the beams deflected at the different angles when the beams scan the breast.

An embodiment of the invention will now be describe by way of example only, with reference to the accompanying drawings, in witch:
FIG. 1 is a schematic view of a prior art mammography apparatus;
FIG. 2 is a schematic perspective view of a dual radiation type mammography apparatus according to an embodiment of the present invention;
FIG. 3 is a conception view of the dual radiation type mammography apparatus of FIG. 2;
FIG. 4 is a schematic view illustrating an image of lesion, which is taken and reformed by a dual radiation type mammography apparatus of an embodiment of the present invention; and
FIG. 5 is a flowchart illustrating a breast imaging method using a dual radiation type mammography apparatus of an embodiment of the present invention.

Embodiments of the present invention will be described below in detail with reference to the accompanying drawings.

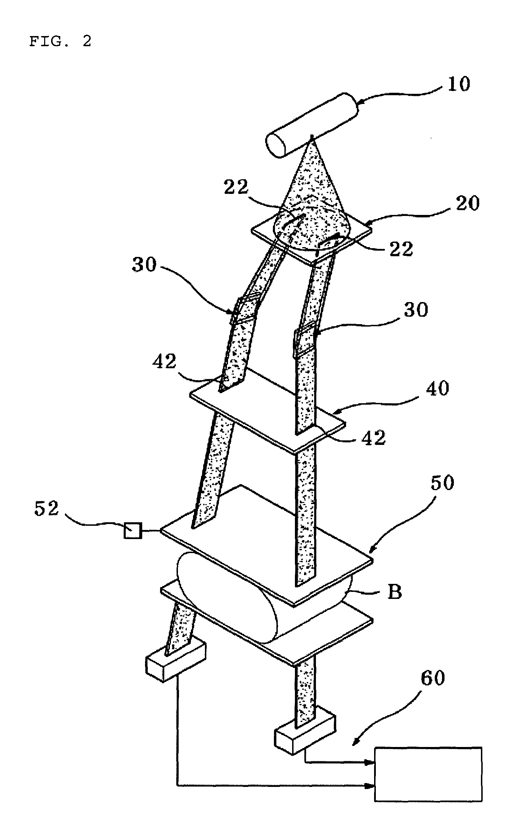

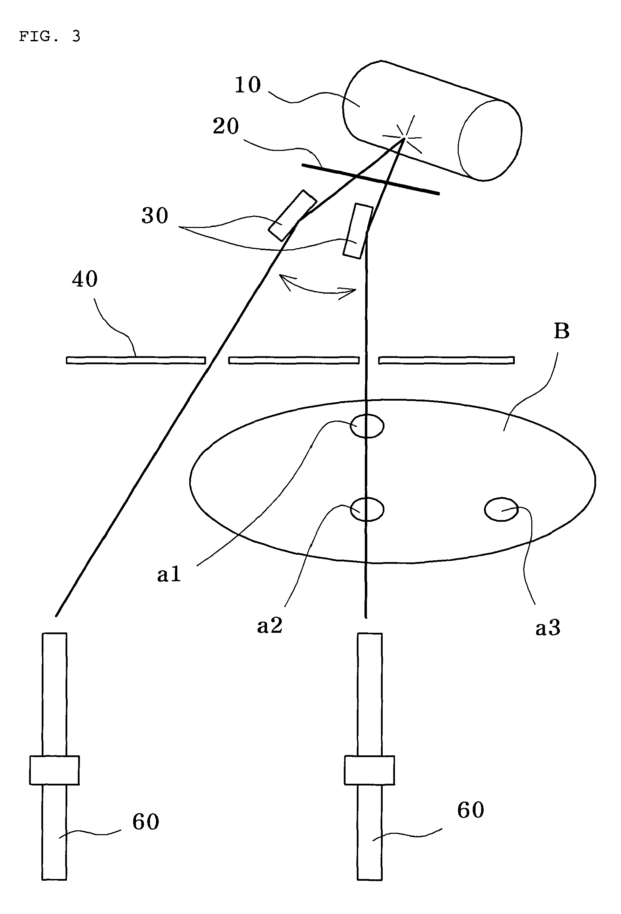

FIG. 2 is a schematic perspective view of a dual radiation type mammography apparatus according to an embodiment of the present invention, and FIG. 3 is a conception view of the dual radiation type mammography apparatus of FIG. 2.

Referring to FIGS. 2 and 3, a mammography apparatus includes a first collimator 20 radiating a plurality of beams from solid angle beams generated from an x-ray generating unit 10, a plurality of multi-layered filter units 30 radiating the plurality of beams from the first collimator 20 at different angles, a second collimator 40 for uniformly maintaining a width of the beams radiated at different angles through the multi-layered filter units 30, a breast fixing unit 50 for fixing the breast B, and an image output unit 60 for reforming breast images taken at the different angles into a three dimensional by scanning the breast B using the beams radiated at the different angles through the second collimator 40.

Like the prior art, the x-ray generating unit 10 uses molybdenum or rhodium as a target material. In addition, since an excessive voltage and excessive current conditions of the x-ray generating unit 10 are identically set to the prior art, the emission spectrum of the x-ray generating unit 10 is identical to that of the prior art x-ray generating unit 10.

The first collimator 20 is provided with a plurality of transmission holes 22 to transmit beams each having a predetermined size among the solid angle beams radiated from the x-ray generating unit 10. The transmission holes 22 may be formed in a fan beam shape so that the beams passing through the transmission holes 22 have the fan beam shape.

The first collimator 20 is formed of lead or tungsten. Some of the solid angle beams pass through the transmission holes 22 (two transmission holes in this embodiment) and the rest is blocked not to be directed to the patient.

The multi-layered filter units 30 filter off some of the beams passing through the first collimator 20 so that beams in a first specific energy band can be deflected at a predetermined angle. The first specific energy band may be 15-23keV. The multi-layered filter units 30 may be formed of molybdenum, rhodium, or aluminum. The multi-layered filter units 30 filter off the beams in the low energy band to reduce the exposure dose to the patient.

In this case, one of the beams (two beams) radiated at different angles from the multi-layered filter units 30 at different angles may be vertically incident on the breast and another (the other) of the beams (two beams) may be incident on the breast at an inclined angle with respect to the vertical line.

The second collimator 40 serves to uniformly maintain a width of the beams that are radiated from the multi-layered filter units 30 at the different angles and emitted to the breast B. The second collimator 40 is formed of a material same as that of the first collimator 20.

The second collimator 40 is provided with filtering holes 42 each having a size identical to that of each pixel of the image detecting unit 60. Therefore, beams in a specific energy band can pass through the filtering holes 42 and the rest is blocked. Therefore the exposure dose to the patient can be significantly reduced. A width of the filtering hole may be 40-60 µm, preferably 50 µm.

By the above-described structure, the fan beams passing through the multi-layered filter units 30 are not straightly directed but incident on the breast B while being diffused in a solid angle shape, thereby preventing the breast B is exposed to ineffective beams and obtaining a high definition image.

The breast fixing unit 50 includes a paddle that holds the breast through which the beams in the second specific energy band are incident. The breast fixing unit 50 is identically structured to the prior art. The breast fixing unit 50 may be formed in a plate shape of a carbon-based material. A pressing plate pressing the breast may be formed of poly methyl meta acrylate or carbon-based material. However, the present invention is not limited to this. Any material that can effectively transmit the beams and minimize an image loss may be used for the breast fixing unit 50.

Specifically, a pressure sensor 52 is provided to the breast fixing unit 50 so as to prevent the breast fixing unit 50 from pressing the breast B with a pressure higher than a predetermined value, thereby minimizing the patient's pain.

The image output unit 60 includes an image capturing unit, an image scanning unit, and an image processing unit. The image capturing unit is arranged in series as long as the width of the beam radiated from the second collimator 40 to form a complete breast image by combining an image obtained before scanning the breast and an image obtained after scanning the breast. The image capturing unit may be a digital detector using a charge coupled device (CCD) or a complementary metal-oxide semiconductor (CMOS) cameral. Therefore, the costs for the components can be minimized and the utilization of the exposure index can be maximized.

The image capturing unit is provided for each of the plurality of the beams. At this point, the image capturing unit may be disposed to be perpendicular to the corresponding beam to increase the resolution of the image.

The image scanning unit is mounted on a motor-driven stage so that the plurality of the fan beams can scan the entire region of the breast. Alternatively, the entire region of the breast may be scanned while moving in a state where the x-rays are fixed.

The image processing unit analyzes numerically the images obtained from the beams radiated vertically or at a predetermined angle through a geometrical reverse and reforms the images into a three-dimensional image so that the examiner can accurately identify the location of the lesion and thus judgment of the examiner can be maximized.

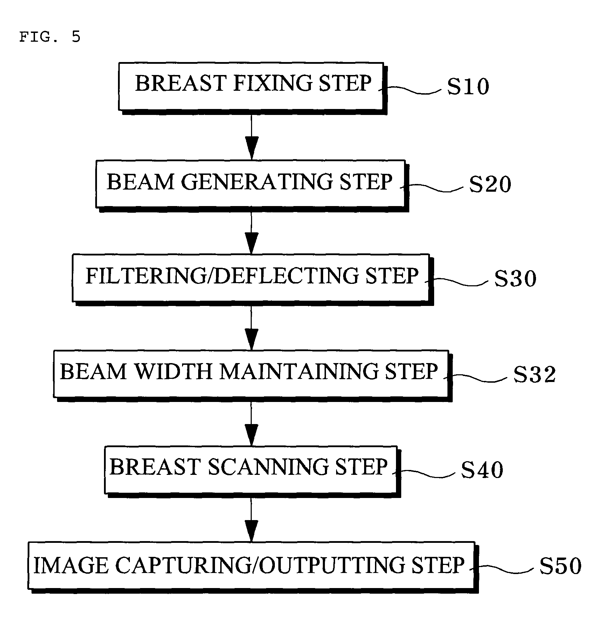

The following will describe a breast imaging method using the dual radiation type mammography apparatus with reference to FIG. 5.

In a breast fixing step S10, the breast B that will be examined is fixed. The breast B is pressed and fixed by the paddle of the breast fixing unit 50. Particularly, since the pressure sensor 52 is provided to the breast fixing unit 50. The pressure applied to the breast B is uniformly maintained by the control of the pressure sensor 52 and thus the patient's pain is minimized.

Needless to say, the pressure sensor 52 may be controlled such that the pressure applied to the breast B can vary. In addition, the x-ray generating unit 10, the first and second collimators 20 and 40, the multi-layered filter units 30, the breast fixing unit 50, and the image output unit 60 are set to be initialized by the motor-driven stage before the breast B is fixed.

In a beam generating step S20, the plurality of beams each having a predetermined size are generated from a solid angle beam generated through the x-ray generating unit. The x-ray generated from the x-ray generating unit 10 is generally the solid angle beam. As the solid angle beam passes through the transmission holes 22 formed on the first collimator 20, the plurality of beams having a predetermined size are formed.

In a filtering/deflecting step S30, a width of each of the beams generated in the beam generating step S30 is adjusted in response to the breast region. In addition, beams in a specific band are incident and deflected at a predetermined angle. That is, the beams passing through the first collimator 20 are incident on the corresponding multi-layered filter units 30.

The multi-layered filter units 30 adjust the width of each of the beams so that the beams can be irradiated to the breast region, and specifically, deflect only the beams in the specific energy band (15-23keV) while filtering the beams in the lower energy band, thereby reducing the exposure dose to the patient. At this point, the multi-layered filter units 30 deflect the beams at different predetermined angles.

Further, one of the beams deflected by the multi-layered filter units 30 is incident on the breast in the vertical direction and the other is incident on the breast B at a predetermined inclined angle with respect to the vertical direction. Therefore, scanned images of the breast B can be taken by the beams incident on the breast B.

In a beam width maintaining step S32, the beams deflected and incident through the filtering/deflecting step S30 are maintained with predetermined widths each corresponding to an image obtainable pixel. That is, the beams deflected by the multi-layered filter units 30 are incident on the second collimator 40 and sized to correspond to a pixel size of the image output unit. In addition, a width of each of the beams directed to the breast B is uniformly maintained.

In a breast scanning step S40, the beams whose widths are uniformly maintained through the beam width maintaining step S32 are incident on the breast to scan the breast. That is, the beams whose widths are uniformly maintained through the second collimator 40 are incident on the breast B fixed by the breast fixing unit 50 to scan the entire region of the breast B.

FIG. 3 is a conception view of the dual radiation type mammography apparatus of FIG. 2.

Referring to FIG. 3, when it is assumed that three lesions a1, a2, and a3 are formed on the breast, in which the lesions a1 and a2 are located on a vertical line and the lesions a3 and a2 is located on a horizontal line, the beams radiated through the second collimator 40 scan the breast B by a width of each fan beam while moving from a side to the other side.

The beam emitted along the vertical line scans the location and vertical section of the lesion a1 and further scans the location and vertical section of the lesion a3 while moving in a direction.

In addition, the beam radiated at an inclined angle with respect to the vertical line scans the section of the breast, which is inclined at a predetermined angle and thus scans the locations and sizes of the lesions a1, a2, and a3.

In an image capturing/outputting step S50, two-dimensional scan images formed by the beams scanning the breast in the breast scanning step S40 are reformed in a three-dimension image to examine if the breast has a lesion. If the breast has the lesion, the size and location of the lesion are output.

That is, the image output unit 60 is disposed on each of the beams scanning the breast B fixed y the breast fixing unit 50 and analyzes numerically the two-dimensional images formed by the respective beams scanning the breast B through the geometrical reverse, thereby reforming the two-dimensional images into the three-dimensional image.

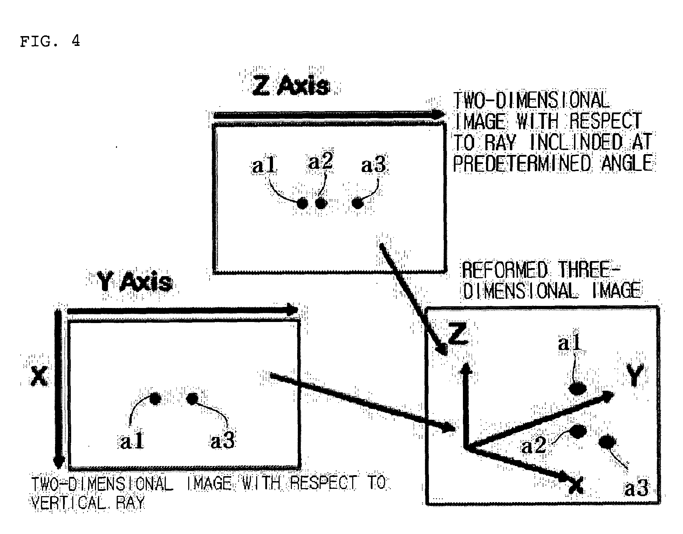

FIG. 4 is a schematic view illustrating an image of lesion, which is taken and reformed by a dual radiation type mammography apparatus of an embodiment of the present invention.

Referring to FIG. 4, a two-dimensional image for the lesions a1 and a2 are captured by the fan beam emitted along the vertical line and a two-dimensional image for the a1, a2, and a3 is captured by the fan beam emitted at an inclined angle. The two-dimensional images formed by the respective beams scanning the breast B through the geometrical reverse, thereby reforming the two-dimensional images into the three-dimensional image. Therefore, the locations and sizes of the lesions can be accurately identified and thus the examiner can accurately describe the examining results and the operation range to the patient.

Specifically, since the image of the breast is taken through only one imaging process, the exposure dose to the patient can be reduced and the patient's pain can be minimized.

According to embodiments of the present invention, since the three dimensional image is formed from two-dimensional images that are captured by the fan beams formed by beams radiated from the-ray generating unit and emitted at different angles the geometrical reverse, the locations and sizes of the lesions can be accurately identified. Therefore, the data collection for maximizing the treatment of the patient becomes possible. In addition, since the breast image taking time is reduced, the x-ray exposure dose to the patient and the patient's pain can be minimized.

The invention is not restricted to the features of the described embodiments.

## Claims

1. A dual-radiation type mammography apparatus for examining a breast by taking a breast image using x-ray beams, the mammography apparatus comprising:
an x-ray generating unit configured to generate the x-ray beams;
a first collimator provided with transmission holes configured such that a solid angle x-ray beam generated from the x-ray generating unit, in the course of passing through the holes, is changed into a plurality of beams each having a predetermined size;
a plurality of multi-layered filter units configured to filter off some of the beams passing through the holes of the first collimator and to deflect beams in a first specific energy band;
a breast fixing unit configured to fix the breast that will be scanned by the beams deflected by the multi-layered filter units; and
a plurality of image output units provided for the respective beams scanning the breast fixed by the breast fixing unit, the image output units configured to reform two-dimensional images formed by the beams deflected at different angles into a three-dimensional image.

2. The dual-radiation type mammography apparatus of claim 1, further comprising a second collimator configured to maintain uniformly a width of each of the beams when the beams deflected by the multi-layered filter units scan the breast.

3. The dual-radiation type mammography apparatus of claim 1, wherein the breast fixing unit comprises a pressure sensor for uniformly controlling fixing pressure pressing the breast.

4. A breast imaging method for examining a breast by taking a breast image using x-ray beams, the breast imaging method comprising:
fixing the breast by applying predetermined pressure to the breast;
forming a plurality of beams by allowing beams in a specific energy band among the solid angle beam generated from an x-ray generating unit to pass and allowing the rest of the beams to be blocked;
filtering some of the beams passing through the holes of the first collimator and deflecting only beams in a specific energy band at different angles;
scanning the breast using the beams deflected at different angles; and
capturing two-dimensional scan images formed by the beams deflected at the different angles and reforming the two-dimensional scan images into a three-dimensional image through a geometrical calculation.

5. The breast imaging method of claim 4, further comprising maintaining uniformly a width of each of the beams deflected at the different angles when the beams scan the breast.
